Europäisches Patentamt

European Patent Office

Office européen des brevets

(11) Veröffentlichungsnummer : **0 021 446**
**B1**

# (12) EUROPÄISCHE PATENTSCHRIFT

(45) Veröffentlichungstag der Patentschrift :
03.11.82

(21) Anmeldenummer : **80103674.0**

(22) Anmeldetag : **28.06.80**

(51) Int. Cl.³ : **A 61 M 25/00**

(54) **Teilbarer Kurzkatheter aus Kunststoff.**

(30) Priorität : 30.06.79 DE 2926572

(43) Veröffentlichungstag der Anmeldung :
07.01.81 (Patentblatt 81/01)

(45) Bekanntmachung des Hinweises auf die Patenterteilung : 03.11.82 Patentblatt 82/44

(84) Benannte Vertragsstaaten :
**AT BE CH DE FR GB IT LI LU NL SE**

(56) Entgegenhaltungen :
FR A 2 364 665
GB A 1 066 751
US A 3 677 244
US A 4 027 659
US A 4 105 732

(73) Patentinhaber : **Intermedicat GmbH**
**Gerliswilstrasse 45**
**CH-6020 Emmenbrücke (CH)**

(72) Erfinder : **Bühler, Wolfgang, Dr.**
**Ernstbergstrasse 8**
**D-3508 Melsungen (DE)**
Erfinder : **Brunner, Franz Werner**
**Waldstrasse 10**
**D-3582 Felsberg 1 (DE)**
Erfinder : **Schacht, Bodo**
**Weihersgrund 1**
**D-3509 Malsfeld (DE)**

(74) Vertreter : **von Kreisler, Alek, Dipl.-Chem. et al**
**Deichmannhaus am Hauptbahnhof**
**D-5000 Köln 1 (DE)**

## Teilbarer Kurzkatheter aus Kunststoff

Die Erfindung betrifft einen aus Kunststoffen zu fertigenden, teilbaren Kurzkatheter, der als Hilfsvorrichtung zur Einführung eines längeren, flexiblen Katheters in einen Patienten, z.B. in dessen Blutgefäßsystem, dient. Nach der Einführung des längeren Katheters kann der Kurzkatheter aus der Punktionsstelle herausgezogen und in Längsrichtung so getrennt werden, daß er von dem verbleibenden Katheter entfernt werden kann.

Zur Einführung flexibler Katheter, die im Rahmen der parenteralen Ernährung oder bei Zugabe von Medikamenten in einen Patienten als Leitungsorgane für die Zufuhr von Flüssigkeiten dienen, verwendet man häufig Hilfsvorrichtungen, durch die der Katheter an seinen Applikationsort, z.B. eine Vene, im Körper des Patienten gebracht wird.

Ein derartiges Hilfsmittel sind z.B. Metallkanülen, durch deren Lumen nach erfolgter Punktion der Katheterschlauch vorgeschoben werden kann. Ein eleganteres, weil weniger gefährliches Verfahren bedient sich eines kurzen Kunststoffkatheters, in einer Ausführungsform, wie sie allgemein zur kurzzeitigen Überleitung von Infusionslösungen und Blut verwendet werden. Sie bestehen in der Regel aus einem dünnwandigen Kunststoffrohr mit einer in ihrem Lumen liegenden metallischen Punktionskanüle ; beide Elemente haben zur Handhabung bzw. zur Verbindung mit anderen Leitungsorganen jeweils einen Ansatz aus Kunststoffen. Kunststoffkanülen haben gegenüber Metallkanülen den Vorteil, daß der einzuführende Katheter bei falscher oder unvorsichtiger Handhabung nicht beschädigt oder abgeschnitten werden kann.

Da der flexible in situ verbleibende Katheter in den meisten Fällen an einem Ende zur Herstellung von Leitungsverbindungen einen fest verbundenen Katheter Ansatz aufweist, ist es verständlich, daß die Kanüle des Kurzkatheters nicht entfernt werden kann, so daß man gezwungen ist, sie außerhalb des Patienten auf dem Katheter verbleibend zu fixieren.

In der DE-C-2 104 226 ist eine Vorrichtung zur Einführung flexibler Katheter beschrieben, die aus einem mit in Längsrichtung verlaufenden Schwächungslinien (Sollbruchstellen) ausgestatteten Kunststoffführungsrohr mit Ansatz besteht. Mittels der am Ansatz angebrachten Griffplatten kann das Kunststoffrohr entlang der Schwächungslinien aufgerissen und danach vom liegenden Katheter entfernt werden.

Geht man davon aus, daß die Schwächungslinien bei dem beschriebenen Gegenstand sich über die gesamte Länge des Kunststoffrohres erstrecken und daß derartige Kunststoffkanülen zwischen 5 und 10 cm lang sind, ersieht man ohne weiteres, daß die Formung einer Schwächungslinie mit exakt definierter und konstant bleibender Restwanddicke auf die jeweilige Kanülenlänge erhebliche Schwierigkeiten bereitet, sei es, daß man sich bei der Formung des bekannten Spritzgießverfahrens bedient oder daß die Schwächungslinien durch spanende Bearbeitung eines vorgeformten und konstante Wanddicken aufweisenden Kunststoffrohres erzeugt werden. Das Einreiß- und Weiterreißverhalten über die gesamte Länge hängt wesentlich von dieser Restwanddicke ab und kann bei ungleichmäßiger Ausbildung dazu führen, daß der Einreißvorgang entgegen der Absicht schon vor der Katheterspitze zu Ende ist und der Katheter sich eben nicht in der gesamten Länge teilen ließ.

Die Erfindung stellt sich daher die Aufgabe, einen Kurzkatheter zu schaffen, bei dem diese Unsicherheiten eliminiert sind und bei dem man auch ohne Bearbeitungs- und Fertigungsaufwand zur Formung der Schwächungslinien zu einem teilbaren Katheter aus Kunststoff gelangt, der sich zuverlässig in Längsrichtung aufreißen und teilen lässt.

Gelöst wird die Aufgabe durch den erfindungsgemäßen teilbaren Kurzkatheter aus Kunststoff, wie er in den Ansprüchen 1 bis 6 gekennzeichnet ist.

Erfindungsgemäß lassen sich im Extrusionsverfahren hergestellte Kunststoffrohre, die beispielsweise für die vorgesehene Anwendung einen Außendurchmesser bis zu 4 mm bei Wanddicken von 0,2 bis 0,4 mm haben, in Längsrichtung vollständig über ihre gesamte Länge teilen, wenn man ein zur Herstellung der Kunststoffkanüle geeignetes Basispolymer mit einem zweiten normalerweise unverträglicher Polymer modifiziert und diese Mischung nach bekannten Extrusionsverfahren zu röhrenartigen Formteilen extrudiert.

Die erfindungsgemäßen teilbaren Kurzkatheter haben den Vorteil, daß sie sich zuverlässig in Längsrichtung aufreißen und teilen lassen und daß sie sich einfach ohne großen Aufwand herstellen lassen.

Die Menge des modifizierenden Polymeren liegt im Bereich von 0,5 bis 40, vorzugsweise 5 bis 25, Gew.-% und die Menge des Basispolymeren im Bereich von 99,5 bis 60, vorzugsweise 95 bis 75 Gew.-%. Die jeweilige entsprechend dem speziellen Polymeren benötigte Menge läßt sich leicht feststellen.

Das Basispolymere ist mit dem modifizierenden Polymeren normalerweise unverträglich.

Aufgrund ihrer chemischen Eigenschaften, d.h. z.B. ihrer chemischen Inaktivität in Kontakt mit menschlichem Blut oder Gewebe, kommen die folgenden Systeme aus Basispolymer einerseits und modifizierendem Polymeren andererseits als besonders geeignet infrage :

| Basispolymer | modifizierendes Polymer |
|---|---|
| Polyäthylen | Polypropylen |
| Polypropylen | Polyäthylen |

Geeignete Gewichtsanteile sind beispielsweise 95 bis 75 Gew.-% Polypropylen als Basispolymer und 5 bis 25 Gew.-% Polyäthylen einer Dichte zwischen 0,915-0,965 g/cm³ als modifizierendes Polymer.

Ist Polyäthylen als Basispolymer vorgesehen und Polypropylen als modifizierendes Polymer, so können die Mengenanteile für das Basispolymer und das modifizierende Polymer ebenfalls in den oben genannten Bereichen liegen.

Wenn auch diese Polymermischungen wegen ihrer chemischen Inaktivität im Kontakt mit menschlichem Gewebe oder mit Blut als besonders geeignet angesehen werden, lassen sich ohne weiteres aus der Vielzahl der bekannten Polymeren Systeme aus Basispolymer und modifizierendem Polymer in variierender prozentualer Zusammensetzung ableiten, die zu Kapillarschläuchen oder -rohren extrudiert, gleiches oder ähnliches Einreiß- und Trennverhalten aufweisen. Beispielsweise bestehen weitere derartige Mischungssysteme aus

| Basispolymer | modifizierendes Polymer |
|---|---|
| Polypropylen | Polyacrylathomo- und copolymere |
| Polypropylen | Polymethacrylathomo- und copolymere |
| Polypropylen | Polystyrolhomo- und copolymere |
| Polypropylen | Polymethylpenten-1 |
| Polypropylen | Polybuten-1 |
| Polypropylen | Polyäthylencopolymerisate |
| Polyäthylen | Polypropylencopolymerisate |
| Polyäthylen | Polybuten-1 |
| Polyäthylen | Polymethylpenten-1 |
| Polyäthylen | Polyacrylathomo- und copolymerisat |
| Polyäthylen | Polymethacrylathomo- und copolymere |
| Polyäthylen | Polystyrolhomo- und copolymerisate |
| Polyvinylchlorid | Polyäthylenhomo- und copolymere |
| Polyvinylchlorid | Polyäthylenhomo- und copolymere |
| Polyvinylchlorid | Polypropylenhomo- und copolymere |
| Polyvinylchlorid | Polymethylpenten-1 |
| Polyvinylchlorid | Polybuten-1 |

Eine spezielle Ausführungsform eines in Längsrichtung teilbaren Kunststoffrohrs unter Verwendung eines Basispolymers und eines modifizierenden zweiten Polymers sieht vor, daß im Extrusionsverfahren ein Rohr aus dem Basispolymer geformt wird und gleichzeitig in dessen Wand als Teil der Wand mindestens zwei parallel um 180° versetzte längs verlaufende Streifen aus dem modifizierenden Polymer mitgeformt werden. An den Grenzflächen Basispolymer und modifizierendes Polymer ist durch die relative Unverträglichkeit beider Polymerer eine homogene « Verschweißung » reduziert bzw. verhindert.

Die Erfindung wird durch die folgenden Beispiele erläutert.

## Beispiel 1

80 Gew.-teile eines Polypropylenhomopolymerisats in Granulatform der Dichte 0,902 g/cm³ und mit einem Schmelzindex MFI 230/5 von 7 g/10 Min. und 20 Gew.-teile eines Polyäthylenhomopolymerisats in Granulatform mit der Dichte 0,920 g/cm³ und einem Schmelzindex MFI 190/2 von 6 g/10 Min. werden gründlich gemischt und in einem Schneckenextruder aufgeschmolzen, als Strang extrudiert und in einem Granulator zu einem Zylindergranulat zerkleinert.

Aus diesem Granulat wird ein Kapillarschlauch in der Abmessung 1,5 mm iD (innerer Durchmesser)/2,0 mm aD (äußerer Durchmesser) extrudiert. Ein Schlauchabschnitt von ca. 5 cm Länge wird an einem Ende mit einem längs geteilten Katheteransatz derart verbunden, daß die Zugkraft der Verbindung Schlauch/Ansatz in Längsrichtung mindestens 20 N beträgt, ohne daß sich die Verbindung löst. Zur Vervollständigung wird in das Schlauchlumen eine Punktionskanüle mit Kanülenansatz eingeschoben. Verwendet man die fertige Punktionsvorrichtung als Hilfsvorrichtung zur Einführung eines Katheters, wird damit die Vene eines zu behandelnden Patienten punktiert, die Metallkanüle entfernt und ein geeigneter flexibler Katheter durch die liegende Kunststoffkanüle vorgeschoben.

Sobald der Katheter plaziert ist, wird die auf dem Katheter befindliche Kunststoffkanüle zurückgezogen und entfernt. Dazu faßt man die am Katheteransatz vorgesehene Griffplatte zwischen Daumen und Zeigefinger jeder Hand und zieht die Griffplatten nach der Seite, reißt dabei den Schlauch ein und trennt ihn bei weiterem seitlichen Ziehen der Länge nach auf. Die Griffplatten und der längs geteilte Schlauch lassen sich von dem Katheter vollständig entfernen.

## Beispiel 2

90 Gewichtsteile eines Polyäthylenhomopolymerisats in Granulatform mit der Dichte 0,960 g/cm³

**0 021 446**

und einem Schmelzindex MFI 190/2 von 5 g/10 min und 10 Gewichtsteile eines Polypropylencopolymerisats (Copolymerisat mit 2 % Äthylen als Comonomeres) mit der Dichte 0,905 g/cm³ und einem Schmelzindex MFI 230/5 von 3,5 g/10 min werden gemischt und in einem Schneckenextruder aufgeschmolzen, als Strang extrudiert und in einem Granulator zu einem Zylindergranulat zerkleinert.

Aus diesem Granulat wird ein Kapillarschlauch in der Abmessung 1,0 mm iD/1,45 mm aD extrudiert. Ein Schlauchabschnitt von 4 cm Länge wird an einem Ende mit einem längs geteilten Katheteransatz derart verbunden, daß die Zugkraft der Verbindung Schlauch/Ansatz in Längsrichtung mindestens 15 N beträgt, ohne daß sich die Verbindung löst. Zur Vervollständigung wird in das Schlauchlumen eine Punktionskanüle mit Kanülenansatz eingeschoben. Verwendet man die fertige Punktionsvorrichtung als Hilfsvorrichtung zur Einführung eines Katheters, wird damit die Vene eines zu behandelnden Patienten punktiert, die Metallkanüle entfernt und ein geeigneter flexibler Katheter durch die liegende Kunststoffkanüle vorgeschoben.

Sobald der Katheter plaziert ist, wird die auf dem Katheter bebefindliche Kunststoffkanüle zurückgezogen und entfernt. Dazu faßt man die am Katheteransatz vorgesehene Griffplatte zwischen Daumen und Zeigefinger jeder Hand und zieht die Griffplatten nach der Seite, reißt dabei den Schlauch ein und trennt ihn bei weiterem seitlichen Ziehen der Länge nach auf. Die Griffplatten und der längs geteilte Schlauch lassen sich von dem Katheter vollständig entfernen.

Weiterhin wird die Erfindung anhand der Abbildungen erläutert.

Abbildung 1

zeigt einen im Extrusionsverfahren hergestellten Kapillarschlauch, d.h. einen erfindungsgemäßen teilbaren Kurzkatheter ;

Abbildung 2

zeigt einen im Extrusionsverfahren hergestellten erfindungsgemäßen teilbaren Kurzkatheter, in dessen Wand 1 zwei um etwa 180° gegeneinander versetzte, zwischen 0,3 und 1 mm breite Streifen 2 und 2′ in Längsrichtung eingelagert sind ;

Abbildung 3

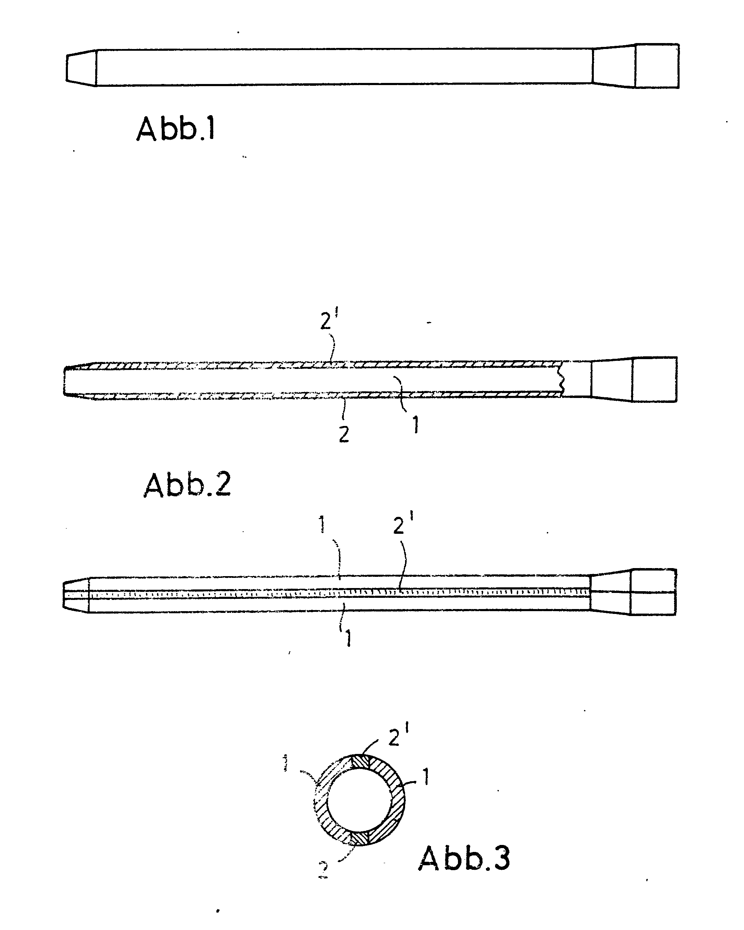

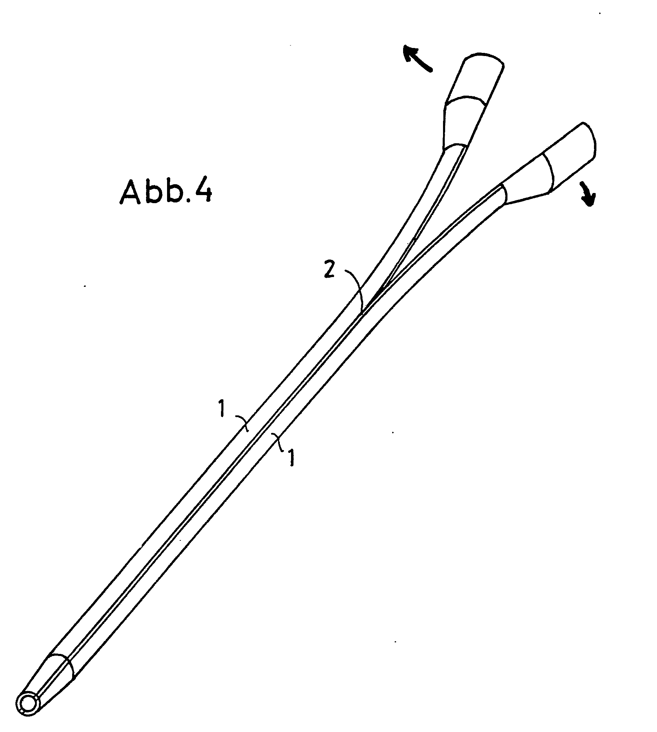

zeigt einen Schnitt durch den Kapillarschlauch der Abb. 2 mit der Wand 1 aus Basispolymer und den als Streifen aus modifizierendem Polymer ausgebildeten Wandabschnitten 2 und 2′ ;

Abbildung 4

zeigt einen teilweise entlang der aus modifizierendem Polymer bestehenden Streifen 2 und 2′ geteilten Katheterschlauch, der durch die als Pfeile dargestellte seitliche, an den Hälften des Katheteransatzes wirkende Kraft getrennt wurde.

### Ansprüche

1. Teilbarer Kurzkatheter aus Kunststoff, dadurch gekennzeichnet, daß er aus einem Basispolymeren und einem normalerweise unverträglichen modifizierenden Polymeren besteht.

2. Katheter nach Anspruch 1, dadurch gekennzeichnet, daß die Menge des modifizierenden Polymeren 0,5 bis 40, vorzugsweise 5 bis 25 Gew.-% und die Menge des Basispolymeren 99,5 bis 60, vorzugsweise 95 bis 75 Gew.-% beträgt.

3. Katheter nach Ansprüchen 1 und 2, dadurch gekennzeichnet, daß das Basispolymer Polypropylen und das modifizierende Polymer Polyäthylen ist.

4. Katheter nach Ansprüchen 1 und 2, dadurch gekennzeichnet, daß das Basispolymer Polyäthylen und das modifizierende Polymer Polypropylen ist.

5. Katheter nach Ansprüchen 1 bis 4, dadurch gekennzeichnet, daß er durch Extrusion eines Gemisches aus Basispolymer und modifizierendem Polymer hergestellt worden ist.

6. Katheter nach Ansprüchen 1 bis 5, dadurch gekennzeichnet, daß die Wand (1) des Katheters aus dem Basispolymer und gleichzeitig ein Teil der Wand in Form von mindestens zwei parallel um 180° versetzten längs verlaufenden Streifen (2 und 2′) aus dem modifizierenden Polymer besteht.

### Claims

1. A divisable short catheter tube made of plastic, characterized in that it consists of a base polymer and a normally incompatible modifying polymer.

2. A catheter according to claim 1, characterized in that the amount of the modifying polymer is 0.5 to 40 percent by weight, and preferably 5 to 25 percent by weight, and the amount of base polymer is 99.5 to 60 percent by weight, and preferably 95 to 75 percent by weight.

3. A catheter according to claims 1 and 2, characterised in that the base polymer is polypropylene and the modifying polymer is polyethylene.

4. A catheter according to claims 1 and 2, characterized in that the base polymer is polyethylene and the modifying polymer is polypropylene.

5. A catheter according to claims 1 through 4, characterized in that it has been manufactured by extrusion of a mixture of the base polymer and the modifying polymer.

6. A catheter according to claims 1 through 5, characterized in that the wall (1) of the catheter tube consists of the base polymer and at the same time a part of the wall having the shape of two longitudinal strips (2 and 2') offset by 180° consists of the modifying polymer.

## Revendications

1. Cathéter court divisible en matière plastique, caractérisé en ce qu'il se compose d'un polymère de base et d'un polymère de modification normalement incompatible.

2. Cathéter selon la revendication 1, caractérisé en ce que la quantité du polymère de modification est comprise entre 0,5 et 40, de préférence entre 5 et 25 % en poids et en ce que la quantité du polymère de base est comprise entre 99,5 et 60, de préférence entre 95 et 75 % en poids.

3. Cathéter selon l'une des revendications 1 ou 2, caractérisé en ce que le polymère de base est du polypropylène et le polymère de modification est du polyéthylène.

4. Cathéter selon l'une des revendications 1 ou 2, caractérisé en ce que le polymère de base est du polyéthylène et en ce que le polymère de modification est du polypropylène.

5. Cathéter selon l'une des revendications 1 à 4, caractérisé en ce qu'il a été fabriqué par extrusion d'un mélange d'un polymère de base et d'un polymère de modification.

6. Cathéter selon l'une des revendications 1 à 5, caractérisé en ce que la paroi (1) du cathéter est formée du polymère de base et en ce que simultanément une partie de la paroi se présente sous la forme d'au moins deux bandes (2, 2') constituées du polymère de modification, orientées longitudinalement et décalées parallèlement de 180°.

Abb.1

Abb.2

Abb.3

Abb.4

2

1

1